# EUROPEAN PATENT SPECIFICATION

(11) **EP 2 220 507 B1**
(45) Date of publication and mention of the grant of the patent: **12.09.2012**
(21) Application number: 08854566.0
(22) Date of filing: 26.11.2008
(51) Int. Cl.: G01N 33/68

(54) **COMPOSITION AND METHOD FOR PREDICTION OF COMPLICATED DISEASE COURSE AND SURGERIES IN CROHN'S DISEASE**
ZUSAMMENSETZUNG UND VERFAHREN ZUR VORHERSAGE EINES KOMPLIZIERTEN KRANKHEITSVERLAUFS UND VON OPERATIONEN BEI MORBUS CROHN
COMPOSITION ET MÉTHODE DE PRÉVISION DES COMPLICATIONS ET DES BESOINS CHIRURGICAUX DANS LA MALADIE DE CROHN

(30) Priority: 30.11.2007 US 5060 P
(43) Date of publication of application: 25.08.2010
(73) Proprietor: Glycominds Ltd., Lod 71291 (IL)
(72) Inventor: DOTAN, Nir, 60850 Shoham (IL); DUKLER, Avinoam, 73127 Moddin (IL)
(74) Representative: Kalhammer, Georg
(86) International application number: PCT/IB2008/003927
(87) International publication number: WO 2009/069007

(56) References cited:
- US-A- 5 004 699
- US-A1- 2004 192 645
- US-A1- 2006 205 014
- US-A1- 2006 205 022
- RIEDER ET AL.: 'The novel anti-glycan antibodies Anti-L and Anti-C in conjunction with ALCA, ACCA, gASCA and AMCA predict early development of fistulae, stenoses and surgery in patients with Crohn's disease: a prospective analysis.' GASTROENTEROLOGY vol. 134, April 2008, page S1, A- 5
- SEOW ET AL.: 'Two novel polysaccharide antibodies (anti-laminarin and anti-chitin) predict an aggressive Crohn's disease phenotype and improve differentiation between Crohn's disease and ulcerative colitis.' GASTROENTEROLOGY vol. 134, April 2008, page S1, A 53

## Description

### Field of the Invention

The invention generally relates to methods of identifying Crohn's disease patients who require surgery and/or who are susceptible to developing complications during the course of disease.

### Background of the Invention

Inflammatory bowel disease (IBD) is a chronic inflammatory disorder of the intestines of unknown etiology. There are two major types of IBD: Crohn's disease (CD) and ulcerative colitis (UC). The diagnosis of IBD is based on a combination of clinical, endoscopic, histopathological, radiologic and laboratory characteristics.

The clinical course of CD is frequently characterized by complications and the need for surgery. A recent study reported that 65-80% of patients with CD undergo at least one surgery during the course of the disease, while many patients require multiple surgical interventions. The complicated disease phenotype is characterized by the presence of fistulae and/or stenosis. About 30% of patients diagnosed with inflammatory disease develop penetrating disease (characterized by abscess, fistula, or phlegmon) within 10 years. The occurrence of complications such as fistulae, stenosis, and the need for surgery cause significant health care burdens.

Therefore, there is a need to identify CD patients who are at risk of requiring surgery within a certain time frame and/or who are susceptible to developing complications repeatedly during the course of the disease. A series of treatment options/levels (e.g., a treatment pyramid) is available for the treatment of CD, wherein more aggressive treatment (a higher level in the pyramid) is utilized for patients with more complicated CD or for patients with the risk of developing complicated CD. In practice, the existence of risk factors, as well as clinical observations, allows physicians to initiate treatment with more aggressive biological agents or to escalate treatment to a higher/more aggressive level, if necessary. Thus, patients identified with a higher risk for disease complications and surgeries are targeted with more aggressive (higher level in the treatment pyramid) therapeutic management.

### Summary of the Invention

In the broadest sense the present application is as defined in the appended claims.

The invention is based, in part, on the determination that anti-laminarin (anti-L) and anti-chitin (anti-C) antibodies serve as prognostic indices for Crohn's disease (CD). Qualitative assessment of the presence of anti-C antibodies in a blood sample, e.g., blood serum of an individual, is useful for risk assessment purposes. The presence of anti-chitin antibodies is correlated with the need for surgical intervention. Assessment of anti-C antibodies has been hampered due to difficulties (e.g., poor solubility) in working with the capture reagent, chitin.

Accordingly, the invention provides methods of predicting a risk for complicated course of CD in a subject. The levels of a variety of antibodies are detected in the blood sample obtained from a subject with CD. The level of an anti-chitin (anti-C) antibody in a blood sample is detected by binding to a carbohydrate reagent comprising an isolated chitosan molecule. The level of an anti-laminarin (anti-L) antibody in the sample is detected by binding to a carbohydrate reagent comprising an isolated laminarin molecule. The level of an anti-mannobioside carbohydrate antibody (AMCA) in the blood sample is detected by binding to a carbohydrate reagent comprising an isolated mannobioside molecule, and the level of an anti-Saccharomyces cerevisiae antibody (gASCA) in the sample is detected by binding to a carbohydrate reagent comprising an isolated mannan. A complicated disease course is predicted by detection of an elevated level of the antibodies in the blood sample relative to a reference level or a control sample from a control population of one or more individuals that do not have CD or have non-complicated CD. Optionally, the reference level is the level under which exists the antibody level of a majority of individuals that do not have CD or have non-complicated CD, e.g., at least about 80-85%; at least about 85-90%; at least about 90-95%; or at least about 95-99% of individuals with antibody levels below the reference level do not have CD or have non-complicated CD. The complicated disease course includes stricturing disease, penetrating disease, or perianal disease. Optionally, an elevated level of the antibodies in the blood sample relative to the reference level or the control sample indicates the subject will need abdominal surgery.

In one aspect, the method of predicting the course of CD in a subject further comprises detecting the levels of additional antibodies in the sample. The level of an anti-laminaribioside carbohydrate antibody (ALCA) in the sample is detected by binding to a carbohydrate reagent comprising an isolated laminaribioside molecule. The level of an anti-chitobioside carbohydrate IgA antibody (ACCA) in the sample is detected by binding to a carbohydrate reagent comprising an isolated chitobioside molecule. A complicated disease course is predicted by detection of an elevated level of the antibodies in the blood sample relative to a reference level or a control sample from a control population of one or more individuals that have do not have CD or have non-complicated CD. Optionally, the reference level is the level under which exists the antibody level, of a majority of individuals that do not have CD or have non-complicated CD, e.g., at least about 80-85%; at least about 85-90%; at least about 90-95%; or at least about 95-99% of individuals with antibody levels below the reference level do not have CD or have non-complicated CD. The complicated disease course includes stricturing disease, penetrating disease, or perianal disease. In one aspect; an elevated level of the antibodies in the blood sample relative to the reference level or the control sample indicates the subject will need abdominal surgery.

Other methods of predicting CD in a subject include detecting a level of an anti-chitin (anti-C) antibody in the blood sample by binding to a carbohydrate reagent comprising an isolated chitosan molecule. A complicated disease course is predicted by detection of an elevated level of the antibody in the blood sample relative to a reference level or a control sample from a control population of one or more individuals that do not have CD or have non-complicated CD. Optionally, the reference level is the level under which exists the antibody level of a majority of individuals that do not have CD or have non-complicated CD, e.g., at least about 80-85%; at least about 85-90%; at least about 90-95%; or at least about 95-99% of individuals with antibody levels below the reference level do not have CD or have non-complicated CD.

Also disclosed is a reagent suitable for predicting the course of CD in a subject, wherein the reagent comprises an isolated chitosan molecule. The isolated chitosan molecule detects a level of an anti-C antibody. A complicated disease course is predicted by detection of an elevated level of the anti-C antibody relative to a reference level or a control sample from a control population of one or more individuals that have non-complicated CD. In one aspect, the isolated chitosan molecule comprises a chitosan lactate molecule. Optionally, the isolated chitosan lactate molecule is associated with a solid substrate. Preferably, the isolated chitosan lactate molecule is associated with the substrate in the absence of a linker.

Reagents suitable for predicting the course of CD in a subject include those that can detect levels of specific antibodies in the subject. The reagent comprises an isolated chitosan molecule, an isolated laminarin molecule, an isolated mannobioside molecule, and an isolated mannan. The isolated chitosan molecule detects a level of an anti-C antibody, while the isolated laminarin molecule detects a level of an anti-L antibody. Similarly, the isolated mannobioside molecule detects a level of an anti-mannobioside carbohydrate antibody (AMCA), while the isolated mannan molecule detects a level of an anti-Saccharomyces cerevisiae antibody (gASCA). A complicated disease course is predicted by detection of an elevated level of the antibodies relative to a reference level or a control sample from a control population of one or more individuals that do not have CD or have non-complicated CD. Optionally, the reference level is the level under which exists the antibody level of a majority of individuals that do not have CD or have non-complicated CD, e.g., at least about 80-85%; at least about 85-90%; at least about 90-95%; or at least about 95-99% of individuals with antibody levels below the reference level do not have CD or have non-complicated CD.

The reagent suitable for predicting the course of CD in a subject may additionally include an isolated chitobioside molecule and an isolated laminaribioside molecule. The isolated chitobioside molecule detects a level of an anti-chitobioside carbohydrate IgA antibody (ACCA), while the isolated laminaribioside molecule detects a level of an anti-laminaribioside carbohydrate antibody (ALCA). A complicated disease course is predicted by detection of an elevated level of the antibodies relative to a reference level or a control sample from a control population of one or more individuals that do not have CD or have non-complicated CD. Optionally, the reference level is the level under which exists the antibody level of a majority of individuals that do not have CD or have non-complicated CD, e.g., at least about 80-85%; at least about 85-90%; at least about 90-95%; or at least about 95-99% of individuals with antibody levels below the reference level do not have CD or have non-complicated CD.

The isolated antigens are purified or synthetic. By "purified" or "substantially purified" is meant a laminarin, chitin, or chitosan molecule or biologically active portion thereof that is substantially free of cellular material or other contaminating macromolecules, e.g., polysaccharides, nucleic acids, or proteins, from the cell or tissue source from which the laminarin, chitin, or chitosan is derived. The phrase "substantially purified" also includes a laminarin, chitin, or chitosan molecule that is substantially free from chemical precursors or other chemicals when chemically synthesized. The language "substantially free of cellular material" includes preparations of laminarin, chitin, or chitosan that are separated from cellular components of the cells from which they are isolated.

Levels of the antibodies are determined by direct or indirect detection. The amounts of the antibody are determined by measuring binding of the antibody to an isolated synthetic or purified molecule or a molecule attached, optionally via a linker, to a solid phase, e.g., a substrate, a plate, or a chip. Optionally, the levels of the antibodies are determined by binding to a carbohydrate reagent comprising an isolated molecule. Alternatively, the amounts of the antibody are determined by measuring binding of the antibody to a polysaccharide containing the antigen.

By "reference level" is meant the mean, median, or range level of each antibody in a population of individuals that do not have CD. Alternatively, the reference level is the mean level plus two standard deviations (SD) of each antibody in a population of one or more individuals that do not have CD. Optionally, the reference level is a qualitative, not a quantitative reference and is expressed in arbitrary units. The greater the relative antibody level compared to the reference level, the greater the risk for the development of complicated CD. For example, if the reference level is about 50 arbitrary units of anti-C antibody, an anti-C antibody level of about 56 arbitrary units would indicate a risk of complications or surgery; an antibody level of about 75 arbitrary units would indicate a high risk of complications or surgery; and an antibody level of about 100 arbitrary units would indicate a very high risk of complications or surgery.

As used herein, the term "specificity" means the probability that a method is negative in the absence of the measured trait. Specificity is calculated as the number of true negative results divided by the sum of the true negatives and false positives. Specificity essentially is a measure of how well a method excludes those who do not have the measured trait. The antibody cut-off value can be selected such that, when the sensitivity is at least about 60%, the specificity of diagnosing/prognosing an individual is in the range of 80-85%, for example, 85-90%, 90-95%, or 95-99%. Optionally, complicated CD is prognosed if the amount of the antibody is greater than the amount of the antibody in a control sample at a cutoff value providing a specificity in the range of 30-60%, for example, at least about 35-60%, 40-60%, 45-60%, or 50-60%. Alternatively, complicated CD is prognosed if the amount of the antibody is greater than the amount of the antibody in a control sample at a cutoff value providing a specificity of at least about 80%, at least about 85%, at least about 90%, or at least about 95%.

The antibodies described herein are useful for assessing the risk of a complicated disease course or the need for surgery in CD patients. The patients may require surgery within at least about 1 month; at least about 3 months; at least about 6 months; at least about 12 months; at least about 18 months; at least about 20 months; at least about 24 months; at least about 40 months; or at least about 80 months. A complicated disease course or a higher risk of the development of complicated CD is predicted/prognosed by detecting elevated levels of at least one of an anti-L antibody and an anti-C antibody. First, a blood sample is provided from a subject with symptoms of CD. Alternatively, a blood sample is provided from a patient diagnosed with CD. The level of an anti-laminarin (anti-L) antibody in the sample is detected by binding to an isolated laminarin molecule. The level of an anti-chitin (anti-C) antibody in the sample is detected by binding to an isolated chitin molecule or a chitosan/chitosan lactate molecule. Optionally, the levels of the antibodies are determined by binding to a carbohydrate reagent comprising an isolated molecule. A complicated disease course is predicted by detection of an elevated level of the antibodies in the blood sample relative to a reference level or a control sample from a control population of one or more individuals that do not have CD or have non-complicated CD, e.g., at least 5%, at least 10%, at least 25%, or at least 50% greater, or at least 2 fold, at least 5 fold, or at least 10 fold greater than a reference level or the amount of the antibody in the control sample. The greater the relative antibody level compared to the reference level, the greater the risk for the development of complicated CD. The complicated disease course includes stricturing disease, penetrating disease, or perianal disease. An elevated level of at least one of an anti-L antibody or an anti-C antibody in the blood sample relative to the reference level or the control sample indicates the subject will need abdominal surgery.

The detection of high levels of an anti-L antibody or an anti-C antibody, alone or in conjunction with gASCA, enables the identification of CD patients who may require surgery within a certain time frame and/or who are susceptible to develop complications and may need to undergo surgery repeatedly during the course of the disease. The patients may require surgery within at least about 1 month; at least about 3 months; at least about 6 months; at least about 12 months; at least about 18 months; at least about 20 months; at least about 24 months; at least about 40 months; or at least about 80 months. These patients should be targeted for more aggressive therapeutic management.

A complicated disease course or a higher risk of the development of complicated CD is predicted/prognosed by detecting elevated levels of at least one of an anti-L antibody and an anti-C antibody, and one or more of an anti-Saccharomyces cerevisiae antibody (gASCA), an anti-laminaribioside carbohydrate antibody (ALCA), an anti-chitobioside carbohydrate IgA antibody (ACCA), and an anti-mannobioside carbohydrate antibody (AMCA). More specifically, a complicated disease course or a higher risk of the development of complicated CD is predicted/prognosed by detecting elevated levels of an anti-L antibody, an anti-C antibody, AMCA, and gASCA. Alternatively, a complicated disease course or a higher risk of the development of complicated CD is predicted/prognosed by detecting elevated levels of an anti-L antibody, an anti-C antibody, AMCA, gASCA, ALCA, and ACCA. The level of an anti-laminarin antibody in the sample is detected by binding to an isolated laminarin molecule. The level of an anti-chitin antibody in the sample is detected by binding to an isolated chitin molecule or an isolated chitosan/chitosan lactate molecule. The level of an anti-Saccharomyces cerevisiae antibody (gASCA) in the sample is detected by binding to an isolated mannan. The level of an anti-laminaribioside carbohydrate antibody (ALCA) in the sample is detected by binding to an isolated laminaribioside molecule. The level of an anti-chitobioside carbohydrate IgA antibody (ACCA) in the sample is detected by binding to an isolated chitobioside molecule. The level of an anti-mannobioside carbohydrate antibody (AMCA) in the sample is detected by binding to an isolated mannobioside molecule. Optionally, the levels of the antibodies are determined by binding to a carbohydrate reagent comprising an isolated molecule. A complicated disease course is predicted/prognosed by detection of an elevated level of at least one of anti-L and anti-C, and one or more of gASCA, ALCA, ACCA, or AMCA in the blood sample relative to a reference level or a control sample from a control population of one or more individuals that do not have CD or have non-complicated CD, e.g., at least 5%, at least 10%, at least 25%, or at least 50% greater, or at least 2 fold, at least 5 fold, or at least 10 fold greater than a reference level or the amount of the antibody in the control sample. Optionally, the reference level is the level under which exists the antibody level of a majority of individuals that do not have CD or have non-complicated CD, e.g., at least about 80-85%; at least about 85-90%; at least about 90-95%; or at least about 95-99% of individuals with antibody levels below the reference level do not have CD or have non-complicated CD. The greater the antibody level compared to the reference level, the greater the risk for the development of complicated CD. The complicated disease course includes structuring disease, penetrating disease, or perianal disease. More specifically, a complicated disease course is predicted/prognosed by detection of an elevated level of an anti-L antibody, an anti-C antibody, AMCA, and gASCA. Alternatively, a complicated disease course is predicted/prognosed by detection of an elevated level of an anti-L antibody, an anti-C antibody, AMCA, gASCA, ALCA, and ACCA.

The course of CD or a higher risk of the development of complicated disease is predicted/prognosed by detecting elevated levels of an anti-L antibody. First, a blood sample is provided from a subject with symptoms of CD. The level of an anti-laminarin (anti-L) antibody in the sample is detected by binding to an isolated laminarin molecule. Optionally, the levels of the antibody are determined by binding to a carbohydrate reagent comprising an isolated molecule. A complicated disease course is predicted by detection of an elevated level of the antibody in the blood sample relative to a control sample or reference level from a control population of one or more individuals that do not have CD or have non-complicated CD. Optionally, the reference level is the level under which exists the antibody level of a majority of individuals that do not have CD or have non-complicated CD, e.g., at least about 80-85%; at least about 85-90%; at least about 90-95%; or at least about 95-99% of individuals with antibody levels below the reference level do not have CD or have non-complicated CD.

The course of CD or a higher risk of the development of complicated disease is predicted/prognosed by detecting elevated levels of an anti-C antibody. First, a blood sample is provided from a subject with symptoms of CD. Alternatively, a blood sample is provided from a patient diagnosed with CD. The level of an anti-chitin (anti-C) antibody in the sample is determined by binding to an isolated chitin molecule or an isolated chitosan/chitosan lactate molecule. Optionally, the levels of the antibodies are determined by binding to a carbohydrate reagent comprising an isolated molecule. A complicated disease course is predicted by detection of an elevated level of the antibody in the blood sample relative to a reference level or a control sample from a control population of one or more individuals that do not have CD or have non-complicated CD. Optionally, the reference level is the level under which exists the antibody level of a majority of individuals that do not have CD or have non-complicated CD, e.g., at least about 80-85%; at least about 85-90%; at least about 90-95%; or at least about 95-99% of individuals with antibody levels below the reference level do not have CD or have non-complicated CD.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below.

Other features and advantages of the invention will be apparent from the following detailed description and claims.

### Brief Description of the Drawing

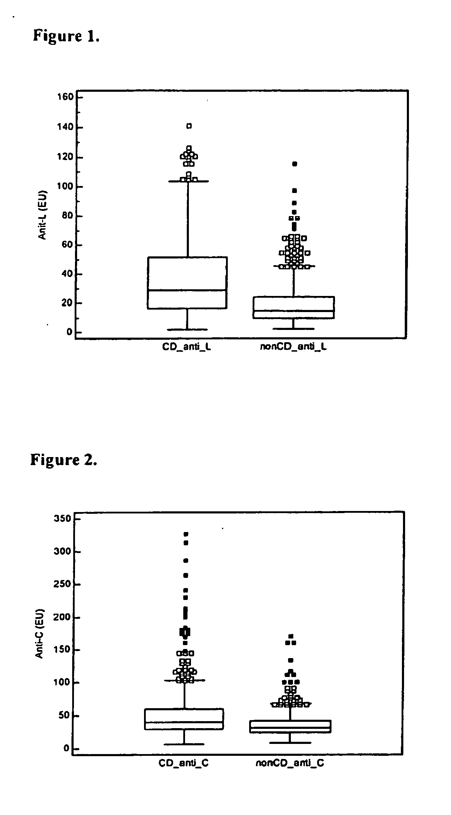

Figure 1 is a graph of box plots showing median values, 25% and 75% quartiles, outside values (unfilled squares), and far out values (filled squares) of Anti-L antibody. Crohn's disease (CD) patients had a significantly higher level of Anti-L than non-CD subjects (p<0.001, Mann Whitney U). EU = arbitrary enzyme immune assay units.
Figure 2 is a graph of box plots showing median values, 25% and 75% quartiles, outside values (unfilled squares), and far out values (filled squares) of Anti-C antibody. CD patients had a significantly higher level of Anti-C than non-CD subjects (p<0.001, Mann Whitney U).
Figure 3 is a graph showing the results of the comparison of anti-Chitin IgA and anti-GIcNAc(b1,4)GIcNAc(b) disaccharide (ACCA) IgA measured in the same sample on the tested cohort. Little correlation was observed between antibodies reactive to chitin (chitosan lactate polysaccharide) and the defined disaccharide fragment glycan ACCA (R2 = 0.15).
Figure 4 is a graph showing the results of the comparison of anti-laminarin IgG and anti-GIc(b1,3)GIc(b) laminaribioside disaccharide (ALCA) IgG. There was no correlation between anti-L (laminarin) IgG and ALCA IgG in a cohort of 208 CD and non CD patients (R2 = 0.15).
Figures 5A to 5F are Kaplan-Meier survival curves describing the difference complications (fistulas or stenosis) free probability between patients that were positive versus negative to: A) ACCA, B) AMCA, C) gASCA, D) anti-C, E) anti-L, and F) patients positive for 2 or more markers versus patients negative to all.
Figures 6A to 6E are Kaplan-Meier survival curves demonstrating the difference surgery free probability between patients that were positive versus negative to: A) AMCA; B) gASCA; C) anti-C; D) anti-L; and E) patients positive for 2 or more markers of AMCA, gASCA, anti-C, and anti L versus patients negative to all.
Figure 7 is a diagram of the structure of chitobioside, wherein "R" represents the remainder of the molecule, e.g., a linker.
Figure 8 is an illustration of the structure of chitin, a polymer of β (1,4)-linked N-acetyl-D-glucosamine, which is non-soluble in water.
Figure 9 is a depiction of the structure of chitosan, a polymer of β (1,4)-linked D-glucosamine, which is soluble in water.
Figure 10 is a diagram of the structure of laminaribioside (GIc(β1,3)GIc(β), wherein "R" represents the rest of the molecule, e.g., a linker.
Figure 11 is an illustration of the structure of laminarin, a polymer of β(1,3) and β(1,6)-linked D-glucose.

### Detailed Description of the Invention

IBDX^{®} is an immunoassay kit containing capture reagents developed and produced by Glycominds Ltd. for the detection of antibodies against the mannan epitope of Saccharomyces cerevisiae (anti-Saccharomyces cerevisiae antibodies; gASCA), laminaribioside (anti-laminaribioside carbohydrate antibodies; ALCA), chitobioside (anti-chitobioside carbohydrate IgA antibodies; ACCA) and mannobioside (anti-mannobioside carbohydrate antibody; AMCA). The intended use of IBDX^{®} is to aid in the diagnosis of CD patients. In conjunction with anti-neutrophil cytoplasmic antibodies (pANCA), IBDX^{®} can be used to aid in IBD diagnosis and the stratification of IBD patients to CD and UC patients. A higher number of antibodies identified with IBDX^{®}, and a higher level of gASCA, ALCA, ACCA or AMCA antibodies is associated with a more complicated CD disease behavior (presence of strictures or fistula), a higher frequency of abdominal surgery and a steroid refractory response (Dotan I et al., 2006 Gastroenterology, 131:366-378; Ferrante M et al., 2007 Gut, 56:1394-1403; and Papp M et al., 2008 Am J Gastroenterol, 103(3):665-81).

As described herein, unexpectedly, in addition to antibodies detected by IBDX^{®}, the level of anti-laminarin (anti-L) IgA and anti-chitin (anti-C) IgA antibodies in a patient's bloodstream (both individually and combined) serves as a risk indicator/assessment and prognostic index for Crohn's disease. Enzyme immune assay (EIA) methods using the antigens laminarin and chitosan lactate were developed for the detection of anti-L and anti-C levels in sera from human patients.

### Chitosan for detection of anti-C antibodies in patient fluid/tissue samples

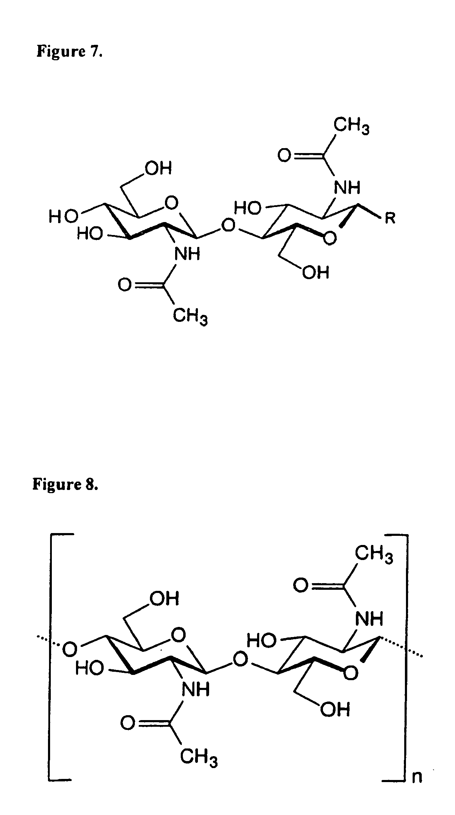

The invention provides different methods of preparing an antigen, e.g., the small molecules described herein, for recognition by an antibody. In order to bind to an antibody, large quantities of the small molecule, e.g., chitobioside (Figure 7), are attached/immobilized to a solid phase via, e.g., a chemical linker. Alternatively, a polymer of the small molecule, e.g., chitin (Figure 8), may be utilized in order to bind to an antibody.

Anti-chitobioside carbohydrate antibodies (ACCA) are associated with complicated Crohn's disease behavior. Because chitobioside (Figure 7) has a low molecular weight (-450 g/mol), it needs to be covalently attached via a linker to a solid phase in order to be recognized by an antibody. Attachment via a linker to a solid phase allows for the stereo-flexibility of chitobioside, which enables the molecule to bind to antibodies, which are much larger than the small molecule itself. Furthermore, unlike high molecular weight molecules, the relatively little surface area of chitobioside does not allow enough noncovalent interactions with the solid phase in order to be absorbed to the surface.

Prior to this invention, the detection of anti-chitobioside antibodies was performed by covalently attaching p-aminophenyl chitobioside to a solid phase. The solid phase was subsequently exposed to patient sera, and the antigen-specific antibodies bound to the antigens on the surface of the solid phase. The bound antibodies were detected and quantified via secondary labeling. However, the process of linker synthesis and the covalent attachment of the linker to chitobioside is expensive and involves many lengthy, complicated steps. As such, prior to the invention described herein, there was a need for the development of a simpler, quicker process for the identification of chitobioside antibodies.

A suitable alternative to chitobioside may include chitin, a linear polymer of β(1,4)-linked N-acetyl-D-glucosamine composed of chitobioside building blocks. See, Figure 8. However, chitin is insoluble in water and cannot be absorbed to surfaces. As such, chitin cannot be used for the detection of anti-chitobioside antibodies. Since chitin is insoluble, several water soluble derivatives of chitin were examined for their ability to bind to anti-chitin antibody.

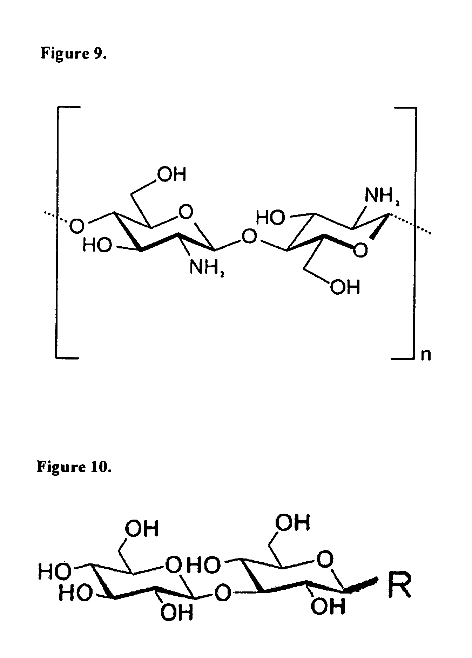

As shown in Figure 9, chitosan is a linear polysaccharide composed of β1,4)-linked D-glucosamine (deacetylated unit). Chitosan is soluble in aqueous solutions and thus, absorbs easily to surfaces. Chitosan has a number of commercial and biomedical uses. Commercial chitosan is derived from the shells of shrimp and other sea crustaceans, and is produced commercially by deacetylation of chitin. Commercially available chitosan is composed not only of β1,4)-linked D-glucosamine (deacetylated unit), but also some randomly distributed N-acetyl-D-glucosamine (acetylated unit) due to incomplete deacetylation. Although some N-acetyl-D-glucosamine monomers may exist in chitosan, it is unclear if the monomers are exposed to the environment or if they would react with anti-chitobioside antibodies. Moreover, as the amine group present on chitosan gives the polymer a high positive charge, it is unknown if antibodies will react with this molecule.

Chitosan lactate is a water-soluble derivative of chitin; however, given the low structure similarity of chitosan lactate and chitin (10%-20%), one skilled in the art of carbohydrate chemistry would not expect that chitosan lactate would bind to anti-chitin antibody. Surprisingly, as described herein, chitosan lactate was best for detection anti-C antibodies. Chitosan lactate is not expressed on the surface of Saccharomyces cerevisiae. Therefore, ASCA does not bind to these molecules. Moreover, anti-C antibodies recognized chitosan in the absence of a chemical linker attaching the small molecule to a solid phase.

The use of chitosan is a significant improvement over prior methods, because it eliminates the need to covalently attach chitobioside to a linker and allows for the absorption of chitosan directly to a solid phase. Chitosan is used for the detection of anti-C antibodies.

### Risk assessment for CD patients

As described herein, the detection of anti-L IgA and anti-C IgA is used as a prognostic tool for CD patients.

As described herein, anti-L and anti-C detection is used to predict the course of CD and is a very important aid for clinicians in determining optimal patient treatment. A positive (determined by cutoff values or reference level) anti-L assay, anti-C assay, or combined anti-L and anti-C assay is associated with a more complicated disease behavior (fistulas or stenosis) and the need for abdominal surgery, while a negative anti-L assay, a negative anti-C assay, or a negative combined anti-L and anti-C assay is associated with the mild inflammatory disease phenotype. Moreover, higher levels of anti-L and anti-C antibodies are also associated with a more complicated disease behavior and the need for abdominal surgery. By contrast, lower levels of anti-L or anti-C antibodies are associated with the mild inflammatory CD phenotype. The antibodies identified by IBDX^{®} are also associated with CD phenotype.

Notably, the sera antibody reactivity to chitosan lactate is different from the reactivity to chitobioside. Additionally, the sera antibody reactivity to laminarin is different from the reactivity to laminaribioside.

The detection of high levels of anti-L, anti-C, alone or in combination with gASCA, enables the identification of CD patients who may require early surgery and/or who are susceptible to develop complications, and may need to undergo surgery early and repeatedly during the course of the disease. These patients should be targeted for more aggressive therapeutic management.

The clinical parameters of sensitivity, specificity, negative predictive value, positive predictive value and efficiency are calculated using true positives, false positives, false negatives and true negatives. A "true positive" sample is a sample positive for CD according to art-recognized methods for diagnosing/prognosing CD, which is also diagnosed positive according to a method of the invention. A "false positive" sample is a sample negative by an art-recognized method, which is diagnosed positive according to a method of the invention. Similarly, a "false negative" is a sample positive for an art-recognized analysis, which is diagnosed negative according to a method of the invention. A "true negative" is a sample negative for the assessed trait by an art-recognized method, and also negative according to a method of the invention. See, for example, Mousy (Ed.), Intuitive Biostatistics New York: Oxford University Press (1995),

As used herein, the term "sensitivity" means the probability that a laboratory method is positive in the presence of the measured trait. Sensitivity is calculated as the number of true positive results divided by the sum of the true positives and false negatives. Sensitivity essentially is a measure of how well a method correctly identifies those with disease. In a method of the invention, the antibody values can be selected such that the sensitivity of diagnosing/prognosing an individual is at least about 20%, and can be, for example, at least about 30%, 40%, 50%, 60%. 70%, 80% or 90%.

As used herein, the term "specificity" means the probability that a method is negative in the absence of the measured trait. Specificity is calculated as the number of true negative results divided by the sum of the true negatives and false positives. Specificity essentially is a measure of how well a method excludes those who do not have the measured trait. The cut-off value can be selected such that, when the sensitivity is at least about 30%, the specificity of diagnosing/prognosing an individual is in the range of 80-99%, for example, 80-85%, 85-90%, 90-95% or 95-99%.

### Example 1: Prognosis of Crohn's Disease Using Anti-L and Anti-C Antibodies

The study population included 1144 clinically well-defined, frozen sera samples from CD patients and controls. Samples taken from patients fulfilling the following requirements were included in the study: Age 18-60 years at time of sampling and definite diagnosis as CD or non CD controls. The present analysis included: (1) Crohn's Disease patients - 549 (52% female, mean age ± SD, 33.4 ± 13.9 years); and (2) Non-CD controls — 595 mean age (SD), (53% female, mean age ± SD,40.7 ± 15.5 years) comprised of: (i) Ulcerative Colitis patients — 333; (ii) irritable Bowel Disease patients — 30; (iii) Indeterminate Colitis patients — 36; (iv) Healthy subjects — 94; and (v) Unaffected CD relatives — 102.

### Anti-L and Anti-C Antibody Detection

For the detection of anti-L antibodies, Nunc plates (Cat. no.445101, Denmark) were activated with cyanuric chloride (Cat. No., SI-C9550-1, Sigma-Aldrich, St Louis, MO, USA) and washed with deionized water. Immediately after washing, wells were filled with 100 µl Laminarin (Cat. No., 109-035-011, Sigma-Aldrich, St Louis, MO, USA), diluted in phosphate-buffered saline (PBS), pH 7.4 (Cat. No. P-3813, Sigma-Aldrich, St Louis, MO, USA) to a final concentration of 100 µg/ml. After overnight incubation at room temperature (RT), plates were washed with deionized water and incubated 15 min with methanol (Cat. No.830108213, Gadot, Israel) and dried at RT.

For the detection of anti-C antibodies, wells were filled with 100 µl chitosan lactate (Cat. No. 523682-1G, Sigma-Aldrich, St Louis, MO, USA) and diluted in PBS to a final concentration of 200 µg/ml. After overnight incubation at RT, plates were washed with deionized water and dried at RT.

Patient's sera were thawed and diluted at 1:101 in IBDX^{™} Sample Diluent (Cat. No. G300023, Glycominds Ltd., Lod, Israel) one day before assay and kept at 4°C until addition to plates. Diluted sera were dispensed 100 µl/well and incubated at RT for 30 min. Plates were washed with Wash Solution (Cat. No. G300022, Glycominds, Lod, Israel) and incubated for 30 min with 100 µl peroxidase conjugated AffiniPure goat anti human sera IgA, a chain specific (Cat. No. 109-035-011, Jackson Immunoresearch, PA, USA) diluted 1:12.500 for Anti-L detection and 1:4000 for Anti-C detection in 50% Stabilizyme HRP (Cat. No. SZ02-2000, SurModics, MN, USA) 50% Water (Cat. No. 03-055-1A, Biological Industries, Israel) to a final concentration of 32 ng/ml for anti-L detection and 0.1 ng/ml for anti-C detection. After 30 minutes incubation at RT, unbound antibodies were removed by washing with Wash Solution. TMB chromogen (Cat. No. RM 1928, Savyon, Israel) was added for 30 minutes and HRP- Stop solution (Cat. No. G300021, Glycominds, Lod, Israel) was added to terminate the enzymatic reaction and absorbance was measured with Victor spectrophotometer (Wallac, Turku, Finland) at 450 nm. A reference sample for each antibody was used to calibrate the antibody levels. Results are reported in arbitrary EIA units (EU). Samples were identified as positive for a particular antibody if the antibody levels were above a reference level. The reference levels for anti-C (90 units) and anti-L (60 units) were set as the upper 95% of the non-CD population.

### Detection of Antibodies with IBDX^{™}

The detection of gASCA, ALCA, ACCA and AMCA with the capture reagents in IBDX^{®} was performed according to the manufacturers instructions (Cat. no. S701100, L703100, C702100 and M704100, Glycominds, Lod, Israel). Results are reported in arbitrary ELISA units (EU). Patients were identified as positive for a particular antibody based on cut-off levels, which were provided by the manufacturer for differentiation between CD and non CD patients.

### Statistical Analysis

The level of anti-C and anti-L was compared between CD to non-CD populations by Mann-Whitney U and box-plots. The association of antibody positivity to clinical phenotype was performed by X² test for nominal data and Mann-Whitney U for continuous data. The association of antibody level to clinical phenotype was performed by Mann-Whitney U. p<0.05 was considered significant. The correlation between anti-polysaccharides and their fragment was calculated using Pearson methods and reported as R2.

### Anti-C IgA and Anti-L IgA Predicts Complicated Disease Course or Surgery in CD Patients

For the CD population two by two tables were created (for X² analyses) according to the following criteria: antibody positivity [positive/negative anti-L, positive/negative anti-C, positive/negative anti-L and/or anti-C (anti-L/C)]; and disease phenotypes (yes or no) for: ileal location (L1), colon location (L2), inflammatory disease behavior (B1), structuring disease behavior (B2), penetrating disease behavior (B3), perianal disease behavior (P), and if the patient had a prior abdominal surgery. In addition, the disease duration, age of diagnosis and number of surgeries were compared according to antibody positivity (positive/negative anti-L, positive/negative anti-C, combined positive/negative anti-L and/or anti-C). Results for antibody positivity, as described below, are shown in **Table 1**.

A significantly higher proportion of CD patients positive for anti-L had penetrating (B3) and perianal (P) disease behavior, and prior abdominal surgery compared to patients negative for anti-L. Also, a significantly higher proportion of CD patients having the mild inflammatory (B1) disease behavior were negative for anti-L compared to patients positive for anti-L. CD patients positive for anti-L also had a longer disease duration and higher number of abdominal surgeries than CD patients negative for anti-L. No association was found between anti-L positivity to age of diagnosis, ileal location (L1), colon location (L2) or to stricturing (B2) phenotypes.

A significantly higher proportion of CD patients that were positive for anti-C had penetrating (B3) and perianal (P) disease behavior and prior abdominal surgery compared to patients negative for anti-C. CD patients positive for anti-C had a lower age of diagnosis and higher number of abdominal surgeries than patients negative for anti-C. No association was found between anti-C positivity to disease duration, ileal location (L1), colon location (L2) or stricturing (B2) disease behavior.

A significantly higher proportion of CD patients positive for the combined anti-L and/or anti-C analysis had penetrating (B3) and perianal (P) disease behavior and prior abdominal surgery than patients negative for the combined analysis. Also, a significantly higher proportion of CD patients negative for the combined anti-L and/or anti-C analysis had the inflammatory (B 1) disease behavior. CD patients positive for the combined analysis had a higher number of abdominal surgeries and longer disease duration than patients negative for the combined analysis. No association was found between the combined anti-L and/or anti-C positivity to ileal location (L1), colon location (L2) or to stricturing (B2) disease behavior.

**Table 2** shows the level of anti-C and anti-L in various CD phenotypes (yes or no) for: inflammatory (B1) disease behavior, stricturing (B2) disease behavior, penetrating (B3) disease behavior, perianal (P) disease behavior and prior abdominal surgery.

**Table 1: Association between positivity of anti-L and anti-C and CD clinical phenotype.**

| | Anti-L (Cutoff 60EU) | | | Anti-C (Cutoff 90 EU) | | | Anti-L and/or Anti-C | | |
|---|---|---|---|---|---|---|---|---|---|
| **Clinical Phenotype** | **Positive** | **Negative** | **p-value Positive vs. Negative** | **Positive** | **Negative** | **p-value Positive vs. Negative** | **Positive** | **Negative** | **p-value Positive vs. Negative** |
| **Number of CD Patients** * | 99 | 450 | | 57 | 492 | | 117 | 432 | |
| **Mean (range) Disease Duration [months]** **s | 147 (1-444) | 123 (0-555) | 0.02 | 148 (1-423) | 125 (0-555) | ns | 145 (1-444) | 122 (0-555) | 0.02 |
| **Mean (range) Age of Diagnosis [yrs]** ** | 19.6 (6-53) | 21.9 (2-76) | ns | 20.4 (8-49) | 21.6 (2-76) | 0.05 | 19.7 (6-53) | 22.0 (2-76) | 0.06 |
| **Ileal Location L1 n (%) †** | 78 (88) | 300 (79.2) | ns | 41 (83.7) | 337 (80.4) | ns | 89 (85.6) | 289 (79.4) | ns |
| **Colon Location L2 n (%) †** | 47 (52.8) | 181 (47.5) | ns | 30 (61.2) | 198 (47.0) | 0.08 | 58 (55.8) | 170 (46.4) | ns |
| **Inflammatory B1 n(%) †** | 34 (38.2) | 196 (50.6) | 0.045 | 17 (34.7) | 213 (49.9) | 0.06 | 40 (38.5) | 190 (51.1) | 0.03 |
| **Stricturing B2 n (%) †** | 26 (29.2) | 121 (31.3) | ns | 13 (26.5) | 134 (31.4) | ns | 32 (30.8) | 115 (30.9) | ns |
| **Penetrating B3 n (%) †** | 31 (31.3) | 89 (19.8) | 0.02 | 23 (40.4) | 97 (19.7) | 0.0007 | 37 (31.6) | 83 (19.2) | 0.006 |
| **Perianal P n (%) †** | 34 (38.2) | 95 (25.2) | 0.02 | 22 (44.9) | 107 (25.7) | 0.007 | 39 (37.5) | 90 (24.9) | 0.02 |
| **Mean Number of Surgeries **** | 1.26 | 1.0 | 0.01 | 1 30 | 10 | 0 02 | 1.3 | 0.99 | 0 005 |
| **Prior Abdominal Surgery n (%) †** | 54 (70.1) | 177 (53 2) | 0.01 | 37 (80.4) | 194 (52.7) | 0.0008 | 64 (71.1) | 167 (52.2) | 0.002 |

| | | | | | | | | | |
|---|---|---|---|---|---|---|---|---|---|
| * Clinical data for each patient was not available for all clinical phenotypes, therefore total n may vary for each phenotype. ** Mann-Whitney U test † Chi-square analysis | | | | | | | | | |

Patients with prior abdominal surgery had higher levels of anti-L than patients without prior abdominal surgery. The level of anti-L was also higher among patients with penetrating (B3) and perianal (P) disease behavior compared to patients without stricturing (B2), penetrating (B3) or perianal (P) disease behavior. Patients with the mild inflammatory disease behavior (B1) had lower levels of Anti-L than patients without the B1 disease behavior. The level of Anti-L was not associated with stricturing disease (B2) behavior.

Patients with prior abdominal surgery had higher levels of anti-C than patients without prior abdominal surgery. The level of anti-C was also higher amongst patients with stricturing (B2), penetrating (B3) and perianal (P) disease behavior than patients without stricturing (B2), penetrating (B3) or perianal (P) diseases behavior. Patients with the mild inflammatory disease behavior (B1) had lower levels of anti-C than patients without the B1 disease behavior.

Patients with prior abdominal surgery had higher levels of gASCA than patients without prior abdominal surgery. The level of gASCA was also higher among patients with structuring (B2) and penetrating (B3) disease behavior compared to patients without stricturing (B2) or penetrating (B3) diseases behavior. Patients with the mild inflammatory disease behavior (B1) had lower levels of gASCA than patients without the B1 disease behavior. The level of gASCA was not associated with perianal disease behavior (P).

Patients with prior abdominal surgery had higher levels of ALCA than patients without prior abdominal surgery. Patients with the mild inflammatory disease behavior (B1) had lower levels of ALCA than patients without the B1 disease behavior. The level of ALCA was not associated with stricturing disease behavior (B2), penetrating disease behavior (B3) or perianal disease behavior (P).

Patients with prior abdominal surgery had higher levels of ACCA than patients without prior abdominal surgery. The level of ACCA was also higher amongst patients with penetrating (B3) or perianal (P) disease behavior compared to patients without B3, or P diseases behavior. Patients with the mild inflammatory disease behavior (B1) had lower levels of ACCA than patients without the B1 disease behavior. The level of ACCA was not associated with stricturing disease behavior (82).

Patients with prior abdominal surgery had higher levels of AMCA than patients without prior abdominal surgery. The level of AMCA was also higher amongst patients with penetrating or perianal (P) disease behavior compared to patients without B3, or P diseases behavior.

Patients with the mild inflammatory disease behavior (B1) had lower levels of AMCA than patients without the B1 disease behavior. The level of AMCA was not associated with stricturing disease behavior (82).

**Table 2: Association between levels of anti-L and anti-C and CD clinical phenotype (Mann-Whitney).**

| Abdominal Surgery Procedure | | | |
|---|---|---|---|
| | No | Yes | P-Value |
| Number Patients | 179 | 231 | |
| Anti-L (mean EU ± SD) | 30.9 ± 25.1 | 43.0 ± 79.4 | <0.0001 |
| Anti-C (mean EU ± SD) | 40.4 ± 27.1 | 60.3 ± 46 | <0.0001 |
| gASCA (mean EU ± SD) | 98.1 ± 48.7 | 92.1 ± 49.5 | <0.0001 |
| ALCA (mean EU ± SD) | 32.9 ± 23.1 | 42.9 ± 29.9 | 0.0006 |
| ACCA (mean EU ± SD) | 35.9 ± 30.7 | 53.1 ± 44.5 | <0.0001 |
| AMCA (mean EU ± SD) | 60.7 ± 49.1 | 68.8 ± 45.8 | 0.01 |
| | | | |

| Olsesse Behavior: Inflammatory B1 | | | |
|---|---|---|---|
| | Yes | No | P-Value |
| Number Patients | 230 | 246 | |
| Anti-L (mean EU ± SD) | 32.9 ± 26.8 | 41.8 ± 28.7 | <0.0001 |
| Anti-C (mean EU ± SD) | 46.4 ± 39.4 | 55.3 ± 37.8 | <0.0001 |
| gASCA (mean EU ± SD) | 61.4 ± 51.0 | 91.3 ± 47.3 | <0.0001 |
| ALCA (mean EU ± SD) | 35.9 ± 26.7 | 41.4 ± 27.9 | 0.01 |
| ACCA (mean EU ± SD) | 41.5 ± 38.7 | 50.5 ± 43.4 | 0.0002 |
| AMCA (mean EU ± SD) | 60.7 ± 49.1 | 67.3 ± 47.4 | 0.05 |
| | | | |

| Disease Behavior: Structuring B2 | | | |
|---|---|---|---|
| | No | Yes | P-Value |
| Number Patients | 329 | 147 | |
| Anti-L (mean EU ± SD) | 37.2 ± 29.9 | 38.1 ± 23.6 | ns |
| Anti-C (mean EU ± SD) | 51.0 ± 42.8 | 51:0 ± 28.0 | 0.03 |
| gASCA (mean EU ± SD) | 71.7± 51.9 | 68.5 ± 48.2 | 0.0002 |
| ALCA (mean EU ± SD) | 37.7 ± 27.2 | 41.2 ± 27.6 | ns |
| ACCA (mean EU ± SD) | 45.5 ± 41.1 | 47.7 ± 42.2 | ns |
| AMCA (mean EU ± SD) | 64.5 ± 46.4 | 64.3 ± 47.0 | ns |
| | | | |

| Disease Behavior Penetrating B3 | | | |
|---|---|---|---|
| | No | Yes | P.Value |
| Number Patients | 429 | 120 | |
| Anti-L (mean EU ± SD) | 34.6 ± 25.4 | 46.0 ± 33.4 | 0.001 |
| Anti-C (mean EU ± SD) | 47.8 ± 36.5 | 61.7 ± 47.3 | 0.0005 |
| gASCA (mean EU ± SD) | 69.3 ± 51.3 | 97.8 ± 47.2 | 0.0001 |
| ALCA (mean EU ± SD) | 38.7 ± 28.0 | 41.4 ± 28.0 | ns |
| ACCA (mean EU ± SD) | 43.7 ± 39.9 | 54.7 ± 48.2 | 0.0007 |
| AMCA (mean EU ± SD) | 62.6 ± 46.2 | 69.3 ± 45.5 | 0.01 |
| | | | |

| Disease Behavior Perianal P | | | |
|---|---|---|---|
| | No | Yes | P-Value |
| Number Patients | 337 | 129 | |
| Anti-L (mean EU ± SD) | 35.4 ± 26.3 | 44.5 ± 31.8 | 0.003 |
| Anti-C (mean EU ± SD) | 46.4 ± 29.5 | 65.1 ± 54.8 | 0.0001 |
| gASCA (mean EU ± SD) | 75.1 ± 51.4 | 83.2 ± 50.3 | 0.09 |
| ALCA (mean EU ± SD) | 38.7 ± 26.7 | 39.1 ± 29.0 | ns |
| ACCA (mean EU ± SD) | 43.0 ± 36.2 | 56.3 ± 46.4 | 0.0001 |
| AMCA (mean EU ± SD) | 61.9 ± 47.5 | 72.4 ± 43.4 | 0.0006 |

### Correlation between levels Anti-C IgA and ACCA IgA or Anti-L IgG and ALCA IgG

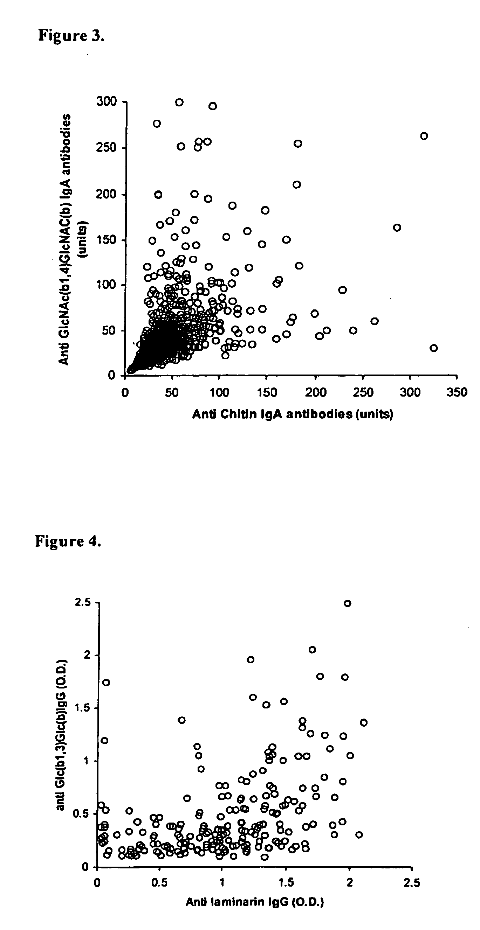

Figure 3 describes the results of the comparison anti-C IgA and ACCA IgA measured in the same sample on the tested cohort. Little correlation was observed between antibodies reactive to chitin (chitosan lactate polysaccharide) and the defined disaccharide fragment glycanGIcNAc(b1,4)GIcNAc(b) disaccharide (ACCA) (R2 = 0.15, Pearson). The results indicate that only 15% of the variance in ACCA is explained by variance in anti-C.

As shown in Figure 8, chitin is a polysaccharide synthesized from units of N-acetylglucosamine (more completely, N-acetyl-D-glucos-2-amine). These units form covalent β1,4 linkages (similar to the linkages between glucose units forming cellulose). Chitin may therefore be described as cellulose with one hydroxyl group on each monomer substituted with an acetylamine group. This allows for increased hydrogen bonding between adjacent polymers, giving the chitin-polymer matrix increased strength. Figure 9 shows that chitosan (a soluble molecule that is structurally similar to chitin) is a linear polysaccharide composed of randomly distributed β(1,4)-linked D-glucosamine (deacetylated unit) and N-acetyl-D-glucosamine (acetylated unit). By contrast, chitobiose is a dimer of β-1,4-linked glucosamine units.

As shown in Figure 4, a lack of correlation and unpredictable nature was observed between antibodies reactive to laminarin (anti-L IgG) and antibodies to the defined disaccharide fragment glycan Glc(b1,3)Glc(b) laminaribioside disaccharide (ALCA) (R2 = 0.17). The results indicate that only 17% of the variance in ALCA is explained by variance in anti-L.

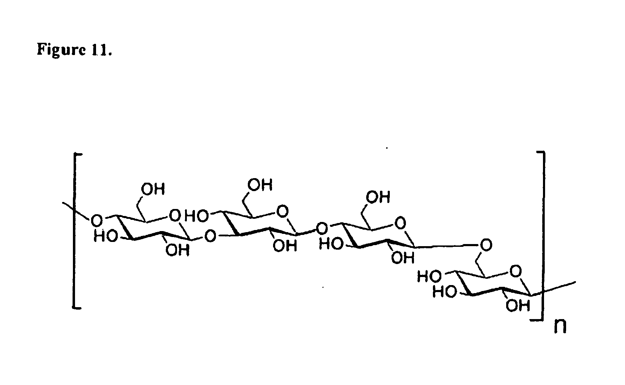

Laminarin (also known as laminaran) is a storage glucan (a polysaccharide of glucose) of Laminaria and other brown algae (Figure 11). It is created by photosynthesis and is made up of β(1,3)-glucans with some β(1,6) linkages and branches. Laminarin is a linear polysaccharide with a β (1, 3):β(1, 6) connectivity in a ratio of 3:1 (Nisizawa et al., 1963 J Biochem, 54:419-426). Although laminaribioside (Figure 10) and laminarin have a common structural element in (Glc(β1,3)Glc(β)), laminarins nevertheless have distinct structural fragments of (Glc(β1,6)Glc(β).

These results demonstrate a lack of correlation (R2 0.15- 0.17) between anti-glycan antibodies reactive to polysaccharides or fragments of the polysaccharides. In both cases there were patients who were highly reactive towards the polysaccharide, but were not reactive towards the defined sub-fragment and vice versa, demonstrating the unpredictable nature of the association. Therefore, one cannot assume that if an individual has developed an immune response to a polysaccharide, he will also react immunologically to a sub-fragment of the polysaccharide.

### Example 2: Anti-L and Anti-C in Combination with ALCA, ACCA, gASCA and AMCA Predict Early Development of Fistulas or Stenosis and Surgery in Patients with Crohn's Disease

Crohn's disease (CD) often leads to complications like fistulae and stenoses, making a surgical intervention necessary. A panel of serological antibodies including the previously unpublished Anti-Laminarin IgA (Anti-L) and Anti-Chitin IgA antibodies were examined for their accuracy in predicting the early appearance of complicated disease or surgery in CD.

Serum samples of 142 CD patients (mean age 33.7 years, 42% female, mean disease duration 52.6 months, procurement 2000-2005) and a control group including samples from 124 ulcerative colitis (UC), 74 other gastrointestinal diseases (OGD) and 249 healthy controls (HC) were tested for the presence of Anti-L, Anti-C, Anti-Chitobioside IgA Carbohydrate Antibodies(ACCA), Anti-Laminaribioside Carbohydrate IgG Antibodies (ALCA), Anti-Mannobioside Carbohydrate IgG Antibodies (AMCA) and Anti-Saccaromyces cervisiae IgG Antibodies (gASCA) by ELISA (Glycominds Ltd., Lod, Israel). No CD patient had fistulas, stenoses or abdominal surgery before or at the time of sample procurement. CD patients were followed up regularly and clinical data were reviewed for the appearance of a complicated disease course or CD related surgery.

### Anti-L and Anti-C Detection

For the detection of anti-L antibodies, Nunc plates (Cat. no.445101, Denmark) were activated with cyanuric chloride (Cat. No., SI-C9550-1, Sigma-Aldrich, St Louis, MO, USA) and washed with deionized water. Immediately after washing, wells were filled with 100 µl Laminarin (Cat. No., 109-035-011, Sigma-Aldrich, St Louis, MO, USA) diluted in phosphate-buffered saline (PBS), pH 7.4 (Cat. No. P-3813, Sigma-Aldrich, St Louis, MO, USA) to a final concentration of 100 µg/ml. After overnight incubation at room temperature (RT), plates were washed with deionized water and incubated 15 min with methanol (Cat. No.830108213, Gadot, Israel) and dried at RT.

For the detection of anti-C antibodies, wells were filled with 100 µl chitosan lactate (Cat. No. 523682-1G. Sigma-Aldrich, St Louis, MO, USA) diluted in PBS to a final concentration of 200 µg/ml. After overnight incubation at RT, plates were washed with deionized water and dried at RT.

Patient's sera were thawed and diluted at 1:101 in IBDX^{™} Sample Diluent (Cat. No. G300023, Glycominds Ltd., Lod, Israel) one day before assay and kept at 4°C until addition toplates. Diluted sera were dispensed 100 µl/well and incubated at RT for 30 min. Plates were washed with Wash Solution (Cat. No. G300022, Glycominds, Lod, Israel) and incubated for 30 min with 100 µl peroxidase conjugated AffiniPure goat anti human sera IgA, α chain specific (Cat. No. 109-035-011, Jackson Immunoresearch, PA, USA) diluted 1:12.500 for Anti-L detection and 1:4000 for Anti-C detection in 50% Stabilizyme HRP (Cat. No. SZ02-2000, SurModics, MN, USA) 50% Water (Cat. No. 03-055-1A, Biological Industries, Israel) to final concentration of 32 ng/ml for anti-L detection and 0.1 ng/ml for anti-C detection. After 30 minutes incubation at RT, unbound antibodies were removed by washing with Wash Solution. TMB chromogen (Cat. No. RM 1928, Savyon, Israel) was added for 30 minutes and HRP-Stop solution (Cat. No. G300021, Glycominds, Lod, Israel) was added to terminate the enzymatic creaction and absorbance was measured with Victor spectrophotometer (Wallac, Turku, Finland) at 450 nm. A reference sample for each antibody was used to calibrate the antibody levels. Results are reported in arbitrary EIA units (EU).

Patients sample were considered positive for anti-C or anti-L if the levels were above a certain cutoff EU value. The cutoff value for determination of positivity for anti- C or anti-L was set based on the upper 95% percentile of a non CD control population.

### Detection of Antibodies with IBDX^{™}

The detection of gASCA, ALCA, ACCA and AMCA with capture reagents in IBDX^{®} was performed according to the manufacturer's instructions (Cat. no. S701100, L703100, C702100 and M704100, Glycominds, Lod, Israel). Results are reported in arbitrary ELISA units (EU). Patients were determined as positive for the antibody if levels were above cutoff value define by the manufacturer.

### Statistical Analysis

Kaplan-Meier estimates, together with log rank tests, Hazard Ratio (HR), and Cox proportional hazard regression models, were used to assess the association between antibodies level (EU and positive status - below or above cutoff) and risk for the development of fistulas or stenosis or having an abdominal surgery.

Median follow-up was 55.4 months (min. 18.2, max. 78.3). 48.6% of the patients developed fistulae or stenoses and 25.3% of the patients had abdominal surgery during follow up. Kaplan-Meier estimates, together with log rank tests and Cox proportional hazard regression models, showed that patients positive for certain anti-glycan antibodies had a significantly higher probability for the appearance of fistulas or stenoses (ACCA, p=0.0072, hazard ratio (HR) 2.0; AMCA p=0.0005, HR 2.4; gASCA p=0.0045, HR 1.9; Anti-L p=0.0002, HR 2.6). In addition, patients positive for AMCA (p=0.045, HR 2.0) or Anti-L (p=0.0007 HR 3.4) had a higher probability of abdominal surgery. CD patients positive for two or more markers had an higher risk for the development of fistulas or stenoses (p=0.005 HR 2.4) and this was also found to be an independent prognostic variable in a Cox regression model corrected for BMI, age, sex, disease duration and small bowel disease location (p=0.008, HR 2.2). CD patients positive for two or more of the antibodies Anti-L, Anti-C, AMCA or gASCA had a higher probability for early surgery (p=0.008, HR 2.8). Moreover, in the Cox regression model this was the only present independent prognostic variable (p<0.001, HR 3.1). The data indicate that the novel anti-glycan antibodies, Anti-L and Anti-C, in conjunction with ALCA, ACCA, gASCA and AMCA, can prospectively predict early development of fistulas or stenosis and surgery in patients with Crohn's disease.

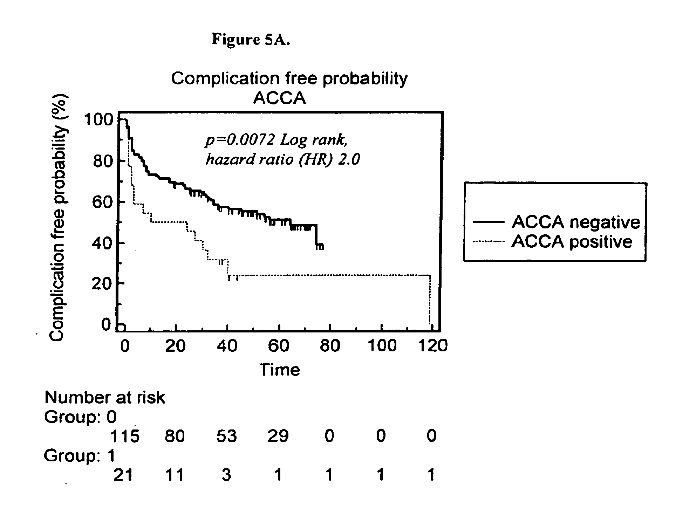

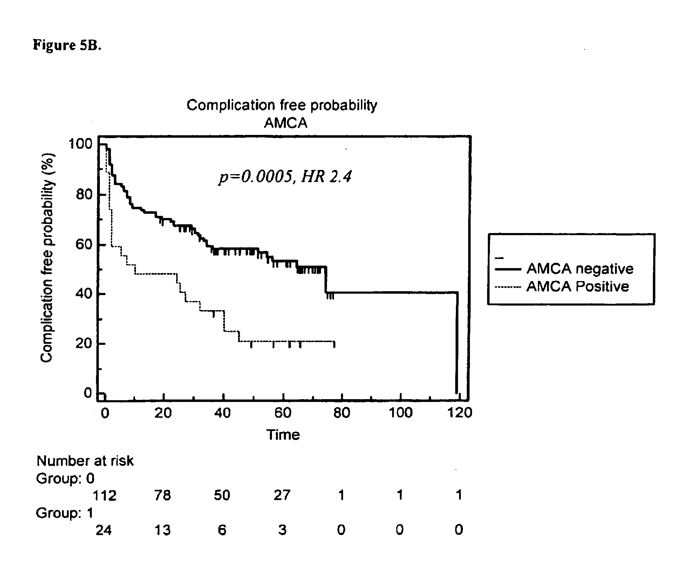

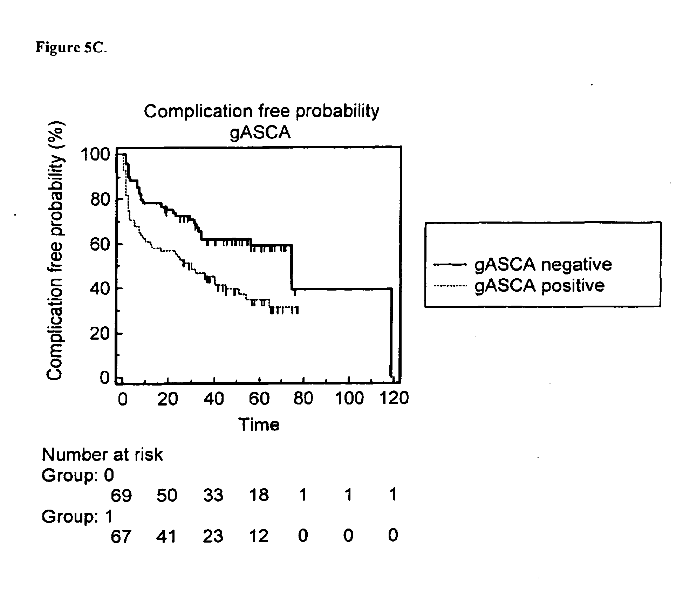

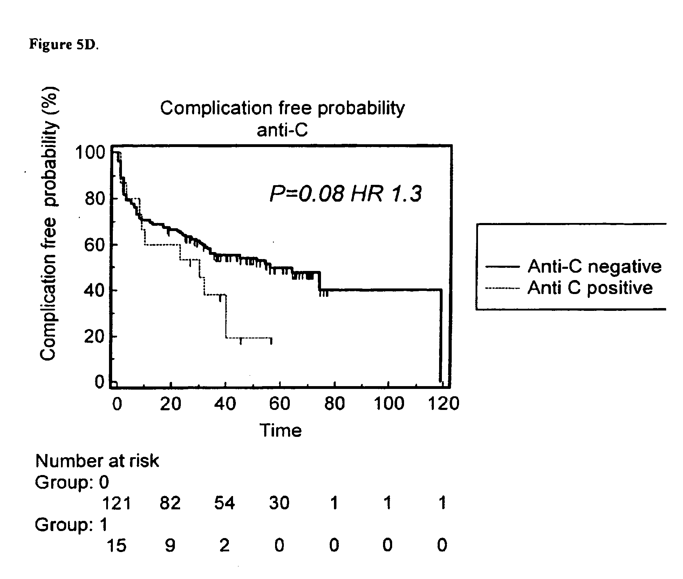

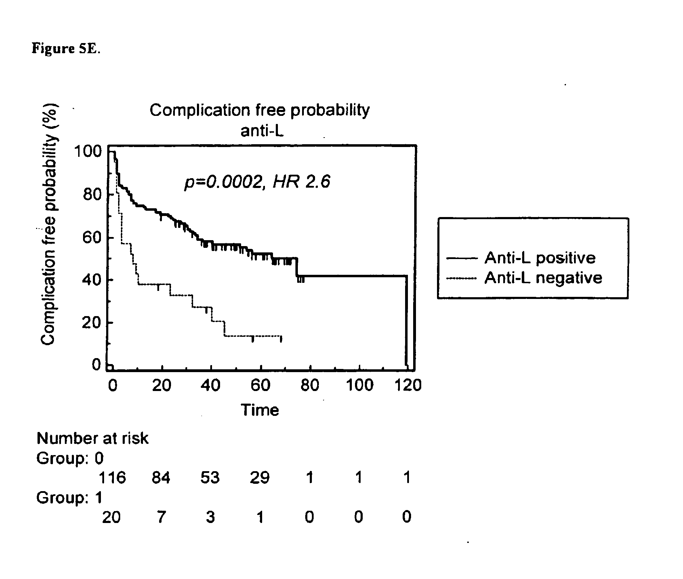

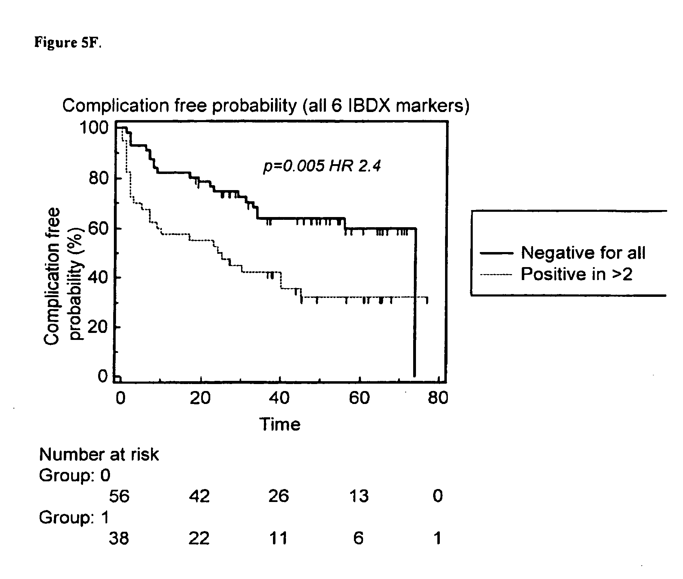

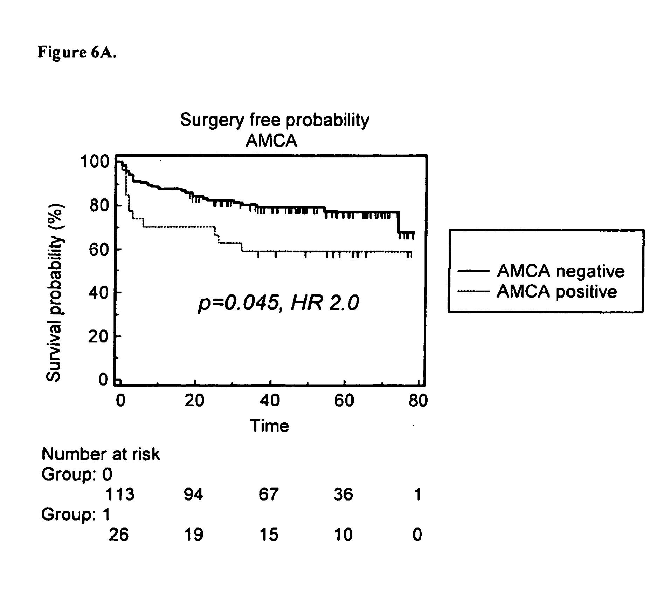

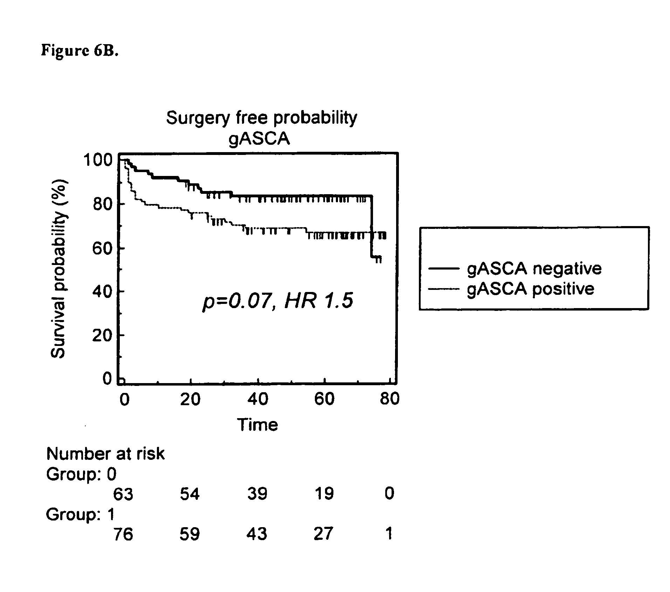

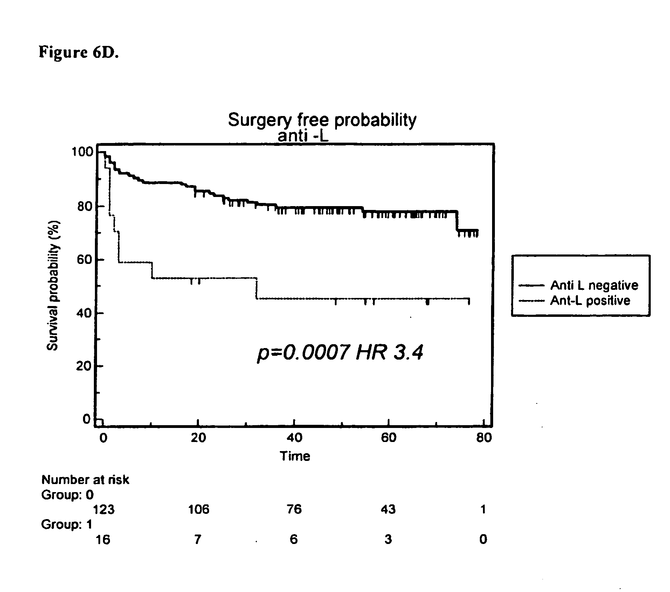

The results of the above-identified experiments are depicted in Figures 5-6, which indicate that the identification of the antibodies described herein at a certain time point inpatients with CD, predict the probability/risk of complicated disease course or surgery within a certain time frame. Figures 5A to 5F are Kaplan-Meier survival curves describing the difference complications (fistulas or stenosis) free probability between patients that were positive versus negative to: A) ACCA, B) AMCA, C) gASCA, D) anti-C, E) anti-L, and F) patients positive for 2 or more markers versus patients negative to all. Figures 6A to 6E are Kaplan-Meier survival curves demonstrating the difference surgery free probability between patients that were positive versus negative to: A) AMCA; B) gASCA; C) anti-C; D) anti-L; and E) patients positive for 2 or more markers of AMCA, gASCA, anti-C, and anti L versus patients negative to all.

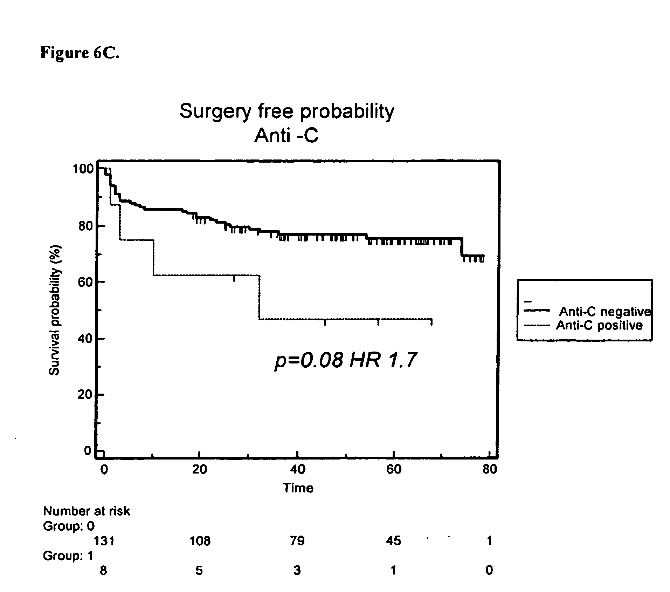

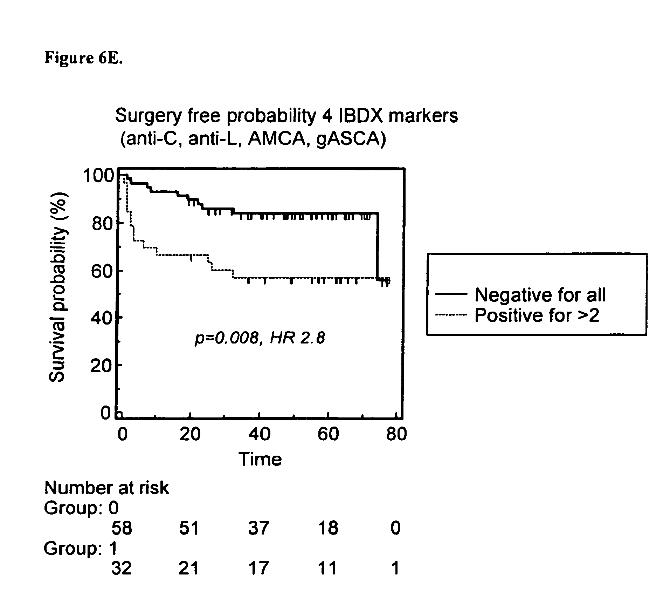

For example, Figure 6C indicates that the probability that a patient positive for anti-C antibodies will not need surgery within 20 months is about 60%, while the probability that a patient negative for anti-C antibodies will not need surgery within 20 months is about 85%. Figure 6E indicates that the probability that a patient positive for at least two of anti-C, anti-L, AMCA, and gASCA will not need surgery within 20 months is about 65%, while the probability that a patient negative for anti-C, anti-L, AMCA, and gASCA will not need surgery within 20 months is about 90%.

## Claims

1. A method of predicting the course of Crohn's disease in a subject, the method comprising:
detecting a level of an anti-laminarin (anti-L) antibody in a blood sample obtained from a subject with Crohn's disease by binding to a carbohydrate reagent comprising an isolated laminarin molecule;
detecting a level of an anti-chitin (anti-C) IgA antibody in said sample by binding to a carbohydrate reagent comprising an isolated chitosan molecule;
detecting a level of an anti-mannobioside carbohydrate antibody (AMCA) in said sample by binding to a carbohydrate reagent comprising an isolated mannobioside molecule;
detecting a level of an anti-Saccharomyces cerevisiae antibody (gASCA) in said sample by binding to a carbohydrate reagent comprising an isolated mannan; and
predicting a complicated disease course by detection of an elevated level of said antibodies in said blood sample relative to a reference level or a control sample from a control population of one or more individuals that have non-complicated Crohn's disease.

2. The method of claim 1, wherein said complicated disease course includes stricturing disease, penetrating disease, or perianal disease.

3. The method of claim 1, wherein an elevated level of said antibodies in said blood samples relative to said reference level or said control sample indicates said subject will need abdominal surgery.

4. The method of claim 1, further comprising:
detecting a level of an anti-laminaribioside carbohydrate antibody (ALCA) in said sample by binding to a carbohydrate reagent comprising an isolated laminaribioside molecule;
detecting a level of an anti-chitobioside carbohydrate IgA antibody (ACCA) in said sample by binding to a carbohydrate reagent comprising an isolated chitobioside molecule; and
predicting a complicated disease course by detection of an elevated level of said antibodies in said blood sample relative to a reference level or a control sample from a control population of one or more individuals that have non-complicated Crohn's disease.

5. The method of claim 4, wherein said complicated disease course includes stricturing disease, penetrating disease, or perianal disease.

6. The method of claim 4, wherein an elevated level of said antibodies in said blood sample relative to said reference level or said control sample indicates said subject will need abdominal surgery.

7. A method of predicting the course of Crohn's disease in a subject, the method comprising:
detecting a level of an anti-chitin (anti-C) IgA antibody in a blood sample obtained from a subject with Crohn's disease by binding to a carbohydrate reagent comprising an isolated chitosan molecule; and
predicting a complicated disease course by detection of an elevated level of said antibody in said blood sample relative to a reference level or a control sample from a control population of one or more individuals that have non-complicated Crohn's disease.

8. A reagent suitable for predicting the course of Crohn's disease in a subject,
wherein said reagent comprises an isolated chitosan molecule, an isolated laminarin molecule, an isolated mannobioside molecule, and an isolated mannan,
wherein said isolated chitosan molecule detects a level of an anti-C antibody in a sample from a subject with Crohn's disease,
wherein said isolated laminarin molecule detects a level of an anti-L antibody in said sample,
wherein said isolated mannobioside molecule detects a level of an anti-mannobioside carbohydrate antibody (AMCA) in said sample,
wherein said isolated mannan molecule detects a level of an anti-Saccharomyces cerevisiae antibody (gASCA) in said sample, and
wherein a complicated disease course is predicted by detection of an elevated level of said antibodies in said sample relative to a reference level or a control sample from a control population of one or more individuals that have non-complicated Crohn's disease.

9. A reagent suitable for predicting the course of Crohn's disease in a subject,
wherein said reagent comprises an isolated chitosan molecule, an isolated laminarin molecule, an isolated mannobioside molecule, an isolated mannan, an isolated chitobioside molecule, and an isolated laminaribioside molecule,
wherein said isolated chitosan molecule detects a level of an anti-C IgA antibody in a sample from a subject with Crohn's disease,
wherein said isolated laminarin molecule detects a level of an anti-L antibody in said sample,
wherein said isolated mannobioside molecule detects a level of an anti-mannobioside carbohydrate antibody (AMCA) in said sample,
wherein said isolated mannan molecule detects a level of an anti-Saccharomyces cerevisiae antibody (gASCA) in said sample,
wherein said isolated chitobioside molecule detects a level of an anti-chitobioside carbohydrate IgA antibody (ACCA) in said sample,
wherein said isolated laminaribioside molecule detects a level of an anti-laminaribioside carbohydrate antibody (ALCA) in said sample, and
wherein a complicated disease course is predicted by detection of an elevated level of said antibodies in said sample relative to a reference level or a control sample from a control population of one or more individuals that have non-complicated Crohn's disease.

## Patentansprüche

1. Verfahren zur Vorhersage des Verlaufs der Crohn-Krankheit bei einem Probanden, wobei das Verfahren:
das Detektieren des Spiegels von Anti-Laminarin- (Anti-L-) -Antikörper in einer Blutprobe, die von einem Probanden mit Crohn-Krankheit erhalten wurde, durch Binden an ein Carbohydratreagens, umfassend ein isoliertes Laminarinmolekül;
das Detektieren des Spiegels von Anti-Chitin- (Anti-C-) -IgA-Antikörper in der Probe durch Binden an ein Carbohydratreagens, umfassend ein isoliertes Chitosanmolekül;
das Detektieren des Spiegels von Anti-Mannobiosid-Carbohydrat-Antikörper (AMCA) in der Probe durch Binden an ein Carbohydratreagens, umfassend ein isoliertes Mannobiosidmolekül;
das Detektieren des Spiegels von Anti-Saccharomyces-cerevisiae-Antikörper (gASCA) in der Probe durch Binden an ein Carbohydratreagens, umfassend ein isoliertes Mannan, und das Vorhersagen eines komplizierten Krankheitsverlaufs durch Detektion eines erhöhten Spiegels der Antikörper in der Blutprobe in Bezug auf einen Referenzspiegel oder eine Kontrollprobe von einer Kontrollpopulation eines oder mehrerer Individuen ohne komplizierte Crohn-Krankheit
umfasst.

2. Verfahren nach Anspruch 1, wobei der komplizierte Krankheitsverlauf stenosierende Erkrankung, penetrierende Erkrankung oder perianale Erkrankung einschließt.

3. Verfahren nach Anspruch 1, wobei ein erhöhter Spiegel der Antikörper in der Blutprobe in Bezug auf den Referenzspiegel oder die Kontrollprobe anzeigt, dass der Proband Abdominalchirurgie benötigen wird.

4. Verfahren nach Anspruch 1, ferner umfassend:
das Detektieren des Spiegels von Anti-Laminaribiosid-Carbohydrat-Antikörper (ALCA) in der Probe durch Binden an ein Carbohydratreagens, umfassend ein isoliertes Laminaribiosidmolekül;
das Detektieren des Spiegels von Anti-Chitobiosid-Carbohydrat-IgA-Antikörper (ACCA) in der Probe durch Binden an ein Carbohydratreagens, umfassend ein isoliertes Chitobiosidmolekül, und
das Vorhersagen eines komplizierten Krankheitsverlaufs durch Detektion eines erhöhten Spiegels der Antikörper in der Blutprobe in Bezug auf einen Referenzspiegel oder eine Kontrollprobe von einer Kontrollpopulation eines oder mehrerer Individuen ohne komplizierte Crohn-Krankheit.

5. Verfahren nach Anspruch 4, wobei der komplizierte Krankheitsverlauf stenosierende Erkrankung, penetrierende Erkrankung oder perianale Erkrankung einschließt.

6. Verfahren nach Anspruch 4, wobei ein erhöhter Spiegel der Antikörper in der Blutprobe in Bezug auf den Referenzspiegel oder die Kontrollprobe anzeigt, dass der Proband Abdominalchirurgie benötigen wird.

7. Verfahren zur Vorhersage des Verlaufs der Crohn-Krankheit bei einem Probanden, wobei das Verfahren:
das Detektieren des Spiegels von Anti-Chitin- (Anti-C-) -IgA-Antikörper in einer Blutprobe, die von einem Probanden mit Crohn-Krankheit erhalten wurde, durch Binden an ein Carbohydratreagens, umfassend ein isoliertes Chitosanmolekül, und
das Vorhersagen eines komplizierten Krankheitsverlaufs durch Detektion eines erhöhten Spiegels des Antikörpers in der Blutprobe in Bezug auf einen Referenzspiegel oder eine Kontrollprobe von einer Kontrollpopulation eines oder mehrerer Individuen ohne komplizierte Crohn-Krankheit
umfasst.

8. Reagens, das zur Vorhersage des Verlaufs der Crohn-Krankheit bei einem Probanden geeignet ist,
wobei das Reagens ein isoliertes Chitosanmolekül, ein isoliertes Laminarinmolekül, ein isoliertes Mannobiosidmolekül und ein isoliertes Mannan umfasst,
wobei das isolierte Chitosanmolekül den Spiegel eines Anti-C-Antikörpers in einer Probe von einem Probanden mit Crohn-Krankheit detektiert,
wobei das isolierte Laminarinmolekül den Spiegel eines Anti-L-Antikörpers in der Probe detektiert,
wobei das isolierte Mannobiosidmolekül den Spiegel eines Anti-Mannobiosid-Carbohydrat-Antikörpers (AMCA) in der Probe detektiert,
wobei das isolierte Mannanmolekül den Spiegel eines Anti-Saccharomyces-cerevisiae-Antikörpers (gASCA) in der Probe detektiert und
wobei ein komplizierter Krankheitsverkauf durch Detektion eines erhöhten Spiegels der Antikörper in der Probe in Bezug auf einen Referenzspiegel oder eine Kontrollprobe von einer Kontrollpopulation eines oder mehrerer Individuen ohne komplizierte Crohn-Krankheit vorhergesagt wird.

9. Reagens, das zur Vorhersage des Verlaufs der Crohn-Krankheit bei einem Probanden geeignet ist,
wobei das Reagens ein isoliertes Chitosanmolekül, ein isoliertes Laminarinmolekül, ein isoliertes Mannobiosidmolekül, ein isoliertes Mannan, ein isoliertes Chitobiosidmolekül und ein isoliertes Laminaribiosidmolekül umfasst,
wobei das isolierte Chitosanmolekül den Spiegel eines Anti-C-IgA-Antikörpers in einer Probe von einem Probanden mit Crohn-Krankheit detektiert,
wobei das isolierte Laminarinmolekül den Spiegel eines Anti-L-Antikörpers in der Probe detektiert,
wobei das isolierte Mannobiosidmolekül den Spiegel eines Anti-Mannobiosid-Carbohydrat-Antikörpers (AMCA) in der Probe detektiert,
wobei das isolierte Mannanmolekül den Spiegel eines Anti-Saccharomyces-cerevisiae-Antikörpers (gASCA) in der Probe detektiert,
wobei das isolierte Chitobiosidmolekül den Spiegel eines Anti-Chitobiosid-Carbohydrat-IgA-Antikörpers (ACCA) in der Probe detektiert,
wobei das isolierte Laminaribiosidmolekül den Spiegel eines Anti-Laminaribiosid-Carbohydrat-Antikörpers (ALCA) in der Probe detektiert und
wobei ein komplizierter Krankheitsverkauf durch Detektion eines erhöhten Spiegels der Antikörper in der Probe in Bezug auf einen Referenzspiegel oder eine Kontrollprobe von einer Kontrollpopulation eines oder mehrerer Individuen ohne komplizierte Crohn-Krankheit vorhergesagt wird.

## Revendications

1. Procédé permettant de prédire l'évolution de la maladie de Crohn chez un sujet, le procédé comprenant :
la détection d'un taux d'un anticorps anti-laminarine (anti-L) dans un échantillon de sang obtenu d'un sujet souffrant de la maladie de Crohn par liaison à un réactif glucidique comprenant une molécule isolée de laminarine ;
la détection d'un taux d'un anticorps IgA anti-chitine (anti-C) dans ledit échantillon par liaison à un réactif glucidique comprenant une molécule isolée de chitosane ;
la détection d'un taux d'un anticorps anti-glucide mannobioside (AMCA) dans ledit échantillon par liaison à un réactif glucidique comprenant une molécule isolée de mannobioside ;
la détection d'un taux d'un anticorps anti-Saccharomyces cerevisiae (gASCA) dans ledit échantillon par liaison à un réactif glucidique comprenant un mannane isolé ; et
la prédiction de l'évolution d'une maladie compliquée par détection d'un taux élevé desdits anticorps dans ledit échantillon de sang par rapport à un taux de référence ou un échantillon contrôle provenant d'une population contrôle d'un ou plusieurs individus qui souffrent d'une maladie de Crohn non compliquée.

2. Procédé selon la revendication 1, dans lequel ladite évolution de maladie compliquée comprend une maladie sténosante, une maladie pénétrante ou une maladie périanale.

3. Procédé selon la revendication 1, dans lequel un taux élevé desdits anticorps dans ledit échantillon de sang par rapport au dit taux de référence ou au dit échantillon contrôle indique que ledit sujet nécessitera une chirurgie abdominale.

4. Procédé selon la revendication 1, comprenant en outre :
la détection d'un taux d'un anticorps anti-glucide laminaribioside (ALCA) dans ledit échantillon par liaison à un réactif glucidique comprenant une molécule isolée de laminaribioside ;
la détection d'un taux d'un anticorps IgA anti-glucide chitiobioside (ACCA) dans ledit échantillon par liaison à un réactif glucidique comprenant une molécule isolée de chitobioside ; et
la prédiction de l'évolution d'une maladie compliquée par détection d'un taux élevé desdits anticorps dans ledit échantillon de sang par rapport à un taux de référence ou un échantillon contrôle provenant d'une population contrôle d'un ou plusieurs individus qui souffrent d'une maladie de Crohn non compliquée.

5. Procédé selon la revendication 4, dans lequel ladite évolution de maladie compliquée comprend une maladie sténosante, une maladie pénétrante ou une maladie périanale.

6. Procédé selon la revendication 4, dans lequel un taux élevé desdits anticorps dans ledit échantillon de sang par rapport au dit taux de référence ou au dit échantillon contrôle indique que ledit sujet nécessitera une chirurgie abdominale.

7. Procédé permettant de prédire l'évolution de la maladie de Crohn chez un sujet, le procédé comprenant :
la détection d'un taux d'un anticorps IgA anti-chitine (anti-C) dans un échantillon de sang obtenu d'un sujet souffrant de la maladie de Crohn par liaison à un réactif glucidique comprenant une molécule isolée de chitosane ; et
la prédiction de l'évolution d'une maladie compliquée par détection d'un taux élevé dudit anticorps dans ledit échantillon de sang par rapport à un taux de référence ou un échantillon contrôle provenant d'une population contrôle d'un ou plusieurs individus qui souffrent d'une maladie de Crohn non compliquée.

8. Réactif approprié pour prédire l'évolution de la maladie de Crohn chez un sujet,
où ledit réactif comprend une molécule isolée de chitosane, une molécule isolée de laminarine, une molécule isolée de mannobioside et un mannane isolé,
où ladite molécule isolée de chitosane détecte un taux d'un anticorps anti-C dans un échantillon provenant d'un sujet souffrant de la maladie de Crohn,
où ladite molécule isolée de laminarine détecte un taux d'un anticorps anti-L dans ledit échantillon,
où ladite molécule isolée de mannobioside détecte un taux d'un anticorps anti-glucide mannobioside (AMCA) dans ledit échantillon,
où ladite molécule isolée de mannane détecte un taux d'un anticorps anti-Saccharomyces cerevisiae (gASCA) dans ledit échantillon, et
où une évolution de maladie compliquée est prédite par détection d'un taux élevé desdits anticorps dans ledit échantillon par rapport à un taux de référence ou un échantillon contrôle provenant d'une population contrôle d'un ou plusieurs individus qui souffrent d'une maladie de Crohn non compliquée.

9. Réactif approprié pour prédire l'évolution de la maladie de Crohn chez un sujet,
où ledit réactif comprend une molécule isolée de chitosane, une molécule isolée de laminarine, une molécule isolée de mannobioside, un mannane isolé, une molécule isolée de chitiobioside et une molécule isolée de laminaribioside,
où ladite molécule isolée de chitosane détecte un taux d'un anticorps IgA anti-C dans un échantillon provenant d'un sujet souffrant de la maladie de Crohn,
où ladite molécule isolée de laminarine détecte un taux d'un anticorps anti-L dans ledit échantillon,
où ladite molécule isolée de mannobioside détecte un taux d'un anticorps anti-glucide mannobioside (AMCA) dans ledit échantillon,
où ladite molécule isolée de mannane détecte un taux d'un anticorps anti-Saccharomyces cerevisiae (gASCA) dans ledit échantillon,
où ladite molécule isolée de chitiobioside détecte un taux d'un anticorps IgA anti-glucide chitiobioside (ACCA) dans ledit échantillon,
où ladite molécule isolée de laminaribioside détecte un taux d'un anticorps anti-glucide laminaribioside (ALCA) dans ledit échantillon, et
où une évolution de maladie compliquée est prédite par détection d'un taux élevé desdits anticorps dans ledit échantillon par rapport à un taux de référence ou un échantillon contrôle provenant d'une population contrôle d'un ou plusieurs individus qui souffrent d'une maladie de Crohn non compliquée.
